(19) Europäisches Patentamt
European Patent Office
Office européen des brevets

(11) **EP 4 372 508 A1**

(12) **EUROPEAN PATENT APPLICATION**

(43) Date of publication:
**22.05.2024 Bulletin 2024/21**

(21) Application number: **23207109.2**

(22) Date of filing: **31.10.2023**

(51) International Patent Classification (IPC):
**G05B 23/02** (2006.01)    **G06F 11/00** (2006.01)
**G06Q 10/20** (2023.01)    **G16H 40/40** (2018.01)

(52) Cooperative Patent Classification (CPC):
**G06Q 10/20; G05B 23/0283; G06F 11/00;**
**G16H 40/40**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.11.2022 US 202218057347**

(71) Applicant: **GE Precision Healthcare LLC
Waukesha, WI 53188 (US)**

(72) Inventors:
• **RAMESH, Nithya**
 **560066 Bengaluru (IN)**
• **AERON, Harsha**
 **560066 Bengaluru (IN)**
• **ALPAY, Bulent**
 **Waukesha, 53188 (US)**
• **WIEDMANN, Uwe**
 **Niskayuna, 12309 (US)**
• **HUANG, Jinming**
 **Waukesha, 53188 (US)**

(74) Representative: **Fennell, Gareth Charles
Kilburn & Strode LLP
Lacon London
84 Theobalds Road
London WC1X 8NL (GB)**

(54) **SYSTEM AND METHODS FOR ENABLING REMOTE PLANNED MAINTENANCE OF MEDICAL IMAGING DEVICES**

(57) One or more systems, devices, computer program products and/or computer-implemented methods of use provided herein relate to a process to facilitate remote planned maintenance of medical imaging devices. A system can comprise a memory that stores computer executable components, and a processor that executes the computer executable components stored in the memory, wherein the computer executable components can comprise an input component that receives sensor data from at least one medical device, and a degradation component that utilizes a feature transformation algorithm to determine a degradation score for a planned maintenance task of the at least one medical device based on the sensor data. Additionally, the computer executable components can comprise a planned maintenance component that recommends maintenance for the at least one medical device based on the degradation score.

FIG. 1

EP 4 372 508 A1

**Description**

TECHNICAL FIELD

The disclosed subject matter relates to applications of algorithms in the planned maintenance of medical imaging devices and, more particularly, the application of algorithms to enabling remote planned maintenance of medical imaging devices.

BACKGROUND

There is a growing interest in the use of algorithms and machine learning to improve the cost of maintenance of medical image devices. Conventional practice is to perform periodic planned maintenance for a system or device multiple times during a year, typically performed by a service engineer. Each planned maintenance instance for a system or device can take up to eight hours and can be executed even if a repair is not needed for the particular system or device. Further, there is a desire to reduce machine downtime and labor costs associated with the maintenance of medical imaging devices.

The above-described background relating to the remote planned maintenance of medical imaging devices is merely intended to provide a contextual overview of some current issues and is not intended to be exhaustive. Other contextual information may become further apparent upon review of the following detailed description.

SUMMARY

The following presents a summary to provide a basic understanding of one or more embodiments described herein. This summary is not intended to identify key or critical elements or delineate any scope of the particular embodiments or any scope of the claims. The sole purpose of the summary is to present concepts in a simplified form as a prelude to the more detailed description that is presented later. In one or more embodiments described herein, systems, devices, computer-implemented methods, and/or computer program products that facilitate enabling remote planned maintenance of medical imaging devices are described.

According to an embodiment, a system can comprise a memory that stores computer executable components and a processor that executes the computer executable components stored in the memory. The computer executable components can include an input component, a degradation component and a planned maintenance component. The input component can receive sensor data from at least one medical device. Additionally, the degradation component can utilize a feature transformation algorithm to determine a degradation score for a planned maintenance task of the at least one medical device based on the sensor data. The planned maintenance component can recommend maintenance for the at least one medical device based on the degradation score.

According to another embodiment, a computer-implemented method of enabling remote planned maintenance of medical imaging devices, can comprise receiving, using a processor operatively coupled to memory, sensor data from at least one medical device. Additionally, the computer-implemented method can include utilizing, using the processor, a feature transformation algorithm to determine a degradation score for a planned maintenance task of the at least one medical device based on the sensor data. Further, the computer-implemented method can include recommending, using the processor, maintenance for the at least one medical device based on the degradation score.

According to yet another embodiment, a computer program product for enabling remote planned maintenance of medical imaging devices, the computer program product can comprise a computer readable storage medium having program instructions embodied therewith, the program instructions executable by a processor can cause the processor to receive sensor data from at least one medical device. Additionally, the computer program product can utilize, using the processor, a feature transformation algorithm to determine a degradation score for a planned maintenance task of the at least one medical device based on the sensor data. Further, the computer program product can recommend, using the processor, maintenance for the at least one medical device based on the degradation score.

DESCRIPTION OF THE DRAWINGS

FIG. 1 illustrates a block diagram of an example, non-limiting system that can facilitate evaluating the sensitivity of a tree-based model, in accordance with one or more embodiments described herein.

FIG. 2 illustrates a block diagram of an example, non-limiting system that can facilitate enabling remote planned maintenance of a medical imaging device, in accordance with one or more embodiments described herein.

FIG. 3 illustrates a block diagram of an example, non-limiting system that can facilitate enabling remote planned maintenance of a medical imaging device by performing principal component analysis via the degradation component, in accordance with one or more embodiments described herein.

FIG. 4A illustrates a 3D cluster graph of an example, non-limiting normal operating region and associated failure regions for a medical imaging device, in accordance with one or more embodiments described herein.

FIGS. 4B and 4C illustrate 2D cluster graphs of an example, non-limiting normal operating region and associated failure regions for a medical imaging device, in accordance with one or more embodiments described herein.

FIG. 5A illustrates a 3D cluster graph of an example, non-limiting normal operating region and associated failure regions for a medical imaging device via Kernel PCA, in accordance with one or more embodiments described herein.

FIGS. 5B illustrates 2D cluster graphs of an example, non-limiting normal operating region and associated failure regions for a medical imaging device via Kernel PCA, in accordance with one or more embodiments described herein.

FIGS. 5C and 5D illustrate 2D cluster graphs and associated statistical graphs of an example, non-limiting system that can facilitate enabling remote planned maintenance of a medical imaging device, in accordance with one or more embodiments described herein.

FIG. 6A illustrates a graph of an example, non-limiting normal operating region and associated failure region for a medical imaging device, in accordance with one or more embodiments described herein.

FIG. 6B illustrates a degradation plot and associated component plots indicating the performance of the medical imaging device relative to normal operation, in accordance with one or more embodiments described herein.

FIG. 7 illustrates degradation plots indicating the performance of the medical imaging device relative to normal operation, in accordance with one or more embodiments described herein.

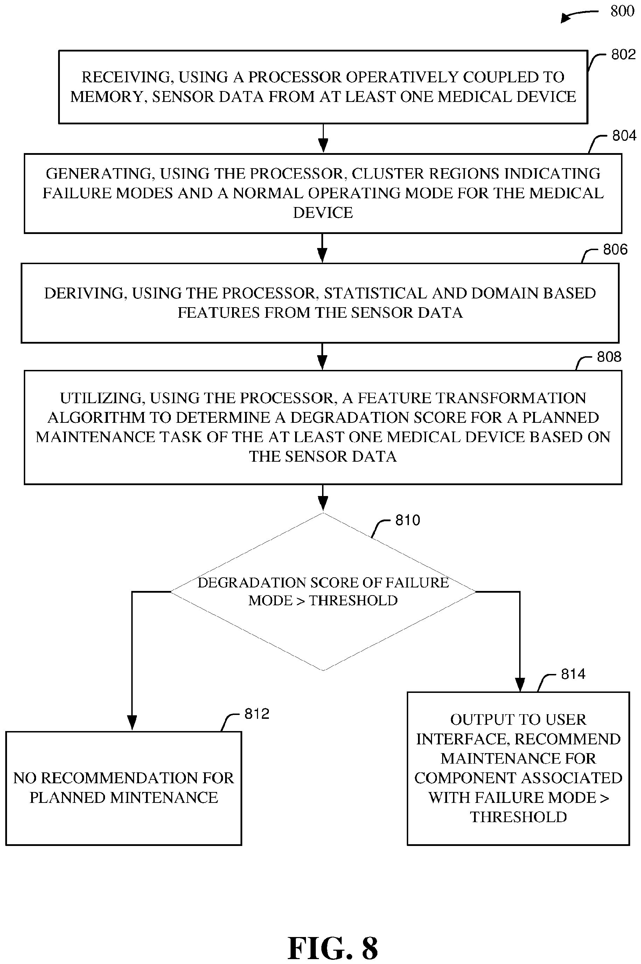

FIG. 8 illustrates a flowchart of an example, non-limiting system that can facilitate enabling remote planned maintenance of a medical imaging device, in accordance with one or more embodiments described herein.

FIG. 9 illustrates an example, non-limiting computing environment in which one or more embodiments described here can be implemented.

FIG. 10 illustrates an example, non-limiting network environment in which one or more embodiments described herein can be implemented.

DETAILED DESCRIPTION

The subject disclosure is now described with reference to the drawings, where like reference numerals are used to refer to like elements throughout. In the following description, for purposes of explanation, numerous specific details are set forth in order to provide a thorough understanding of the subject disclosure. It may be evident, however, that the subject disclosure may be practiced without these specific details. In other instances, well-known structures and devices are shown in block diagram form in order to facilitate describing the subject disclosure. Furthermore, there is no intention to be bound by any expressed or implied information presented in the preceding Background or Summary sections, or in the Detailed Description section.

As illuded to above, planned maintenance can be improved in one or more various ways, and various embodiments are described herein to this end and/or other ends.

In some cases, the task of scheduling and performing planned maintenance for a medical imaging device can include inspecting the system and/or conducting repairs multiple times over a year (e.g., once a year or once every two years), and in some cases, where maintenance may not be required. Generally, maintenance activities can be scheduled at specified intervals (e.g., every 3 months) to care and service non-failed Systems/Subsystems/Components (SSCs) for the purpose of maintaining the medical device in satisfactory operating condition before a failure results in downtime. Due to the complexity of medical devices, planned maintenance can include numerous tasks (e.g., upwards of 100 in some cases) and thus, the downtime of the medical device can be high. At times, maintenance tasks can be performed irrespective of whether a repair or upkeep is actually needed, such systems fail to consider the current state of the medical device (or component included therewith). In examples, a medical device can be scheduled for maintenance, but the medical device may not actually require repair/maintenance. In one or more of a variety of manners, the device can perform longer than the expected performance duration for the device. Such repairs on devices performing to standard are wasteful and costly. Conversely, a repair can be required for a medical device after performance of the medical device degrades to a level of unacceptable performance.

Nonetheless, even though maintenance can be scheduled and performed for medical devices, a problem associated with planned maintenance, is that it can be conducted when a medical device is performing as expected, or after the medical device has degraded below an acceptable level of performance. One or more embodiments described herein can be implemented to provide service engineers the ability to decide whether a scheduled planned maintenance task is really desired or not based on the current state of the SSCs; thus enabling the current practice of on-site planned maintenance to remote planned maintenance.

Given these problems, one or more embodiments described herein can be implemented to produce a solution to one or more of these problems in the form of systems, computer-implemented methods, and/or computer program products that can facilitate the following processes: i) receiving, using a processor operatively coupled to memory, sensor data from at least one medical device; ii) utilizing, using the processor, a feature transformation algorithm to determine a degradation score for a planned maintenance task of the at least one medical device based on the sensor data; and iii) recommending, using the processor, maintenance for the at least one medical device based on the degradation score. That is, embodiments described herein include one or more systems, computer implemented methods, apparatuses and/or computer program products that can facilitate one or more of the aforementioned processes.

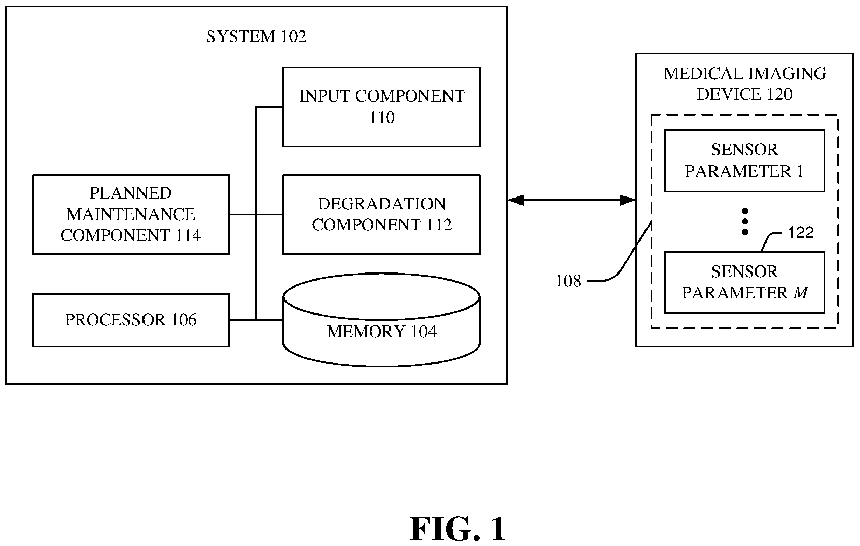

FIG. 1 illustrates a block diagram of an example, non-limiting system 102 that can facilitate enabling remote planned maintenance of a medical imaging device. The non-limiting system 102 can comprise one or more of a variety of components, such as memory 104, processor 106, bus 108, input component 110, degradation component 112, planned maintenance component 114, and one or more medical imaging devices 120 (e.g., which can include one or more varieties of medical devices). Additionally, the input component 110 can receive sensor data from at least one medical imaging device 120. In various embodiments, one or more of memory 104, processor 106, bus 108, input component 110, degradation component 112, planned maintenance component 114, and one or more medical imaging devices 120 can be communicatively or operatively coupled (e.g., over a bus or wireless network) to one another to perform one or more functions of the system 102.

With embodiments, the various components of the remote planned maintenance system 102 can be connected such that the degradation component 112 can utilize a feature transformation algorithm 210 to determine a degradation score for a planned maintenance task of the at least one medical imaging device 120 based on the received sensor data (e.g., via the input component 110). Repetitive description of like elements employed in other embodiments described herein is omitted for sake of brevity. Aspects of systems (e.g., the remote planned maintenance system 102, and the like), apparatuses or processes in various embodiments of the present invention can constitute one or more machine-executable components embodied within one or more machines (e.g., embodied in one or more computer readable mediums (or media) associated with one or more machines). Such components, when executed by the one or more machines (e.g., computers, computing devices, virtual machines, a combination thereof, and/or the like) can cause the machines to perform the operations described.

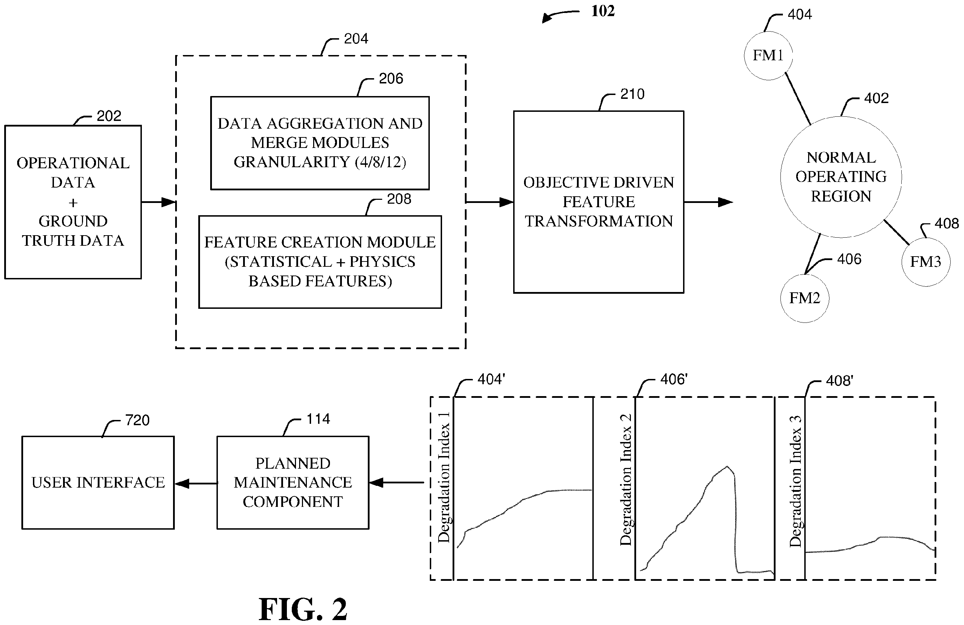

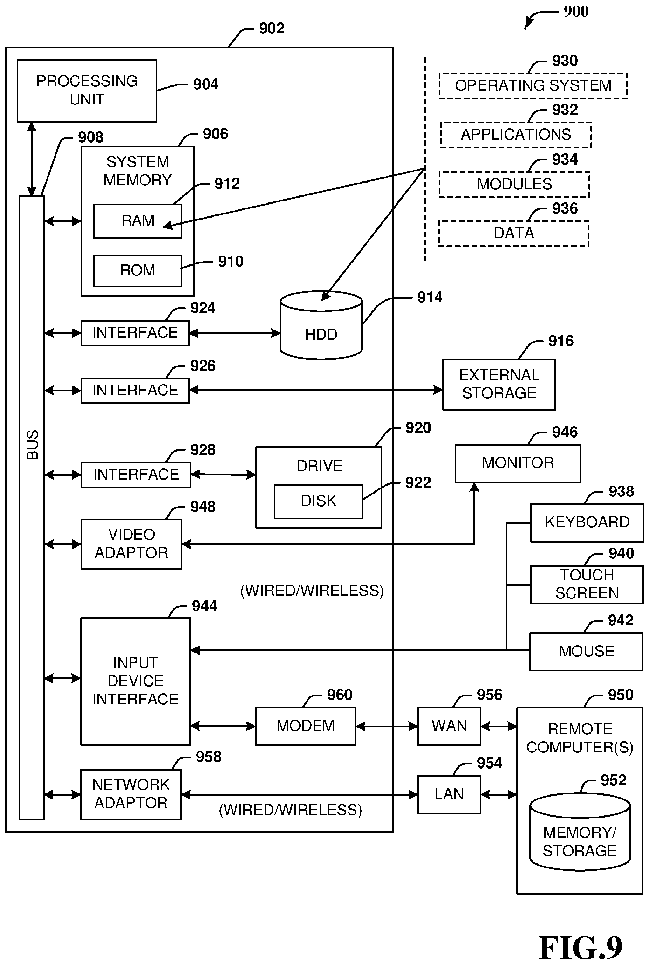

Additional description of functionalities will be further described below with reference to the example embodiments of FIGS. 1 and 2, where repetitive description of like elements and/or processes employed in respective embodiments is omitted for sake of brevity. The remote planned maintenance system 102 can facilitate: i) receiving, using a processor operatively coupled to memory, sensor data from at least one medical imaging device 120; ii) utilizing, using the processor, a feature transformation algorithm 210 to determine a depredation score for a planned maintenance task of the at least one medical imaging device 120 based on the sensor data; and iii) recommending, using the processor, maintenance for the at least one medical imaging device 120 based on the degradation score. The degradation score can further indicate a propensity for a particular failure mode of the at least one medical imaging device 120. Further, the planned maintenance component 114 can include a failure threshold for the planned maintenance task associated with the medical imaging device 120. One or more various sources of information can be utilized in determining the degradation index and/or the failure threshold for the respective medical imaging device 120. Such information can include sensor data obtained directly from the medical imaging device 120 (e.g., raw sensor data) and/or can include performance/part information (e.g., schematic-based information from the manufacturer of the medical imaging device 120) obtained from a linked medical imaging device 120 informational database, which can be associated with operating environment 900 (Fig. 9).

In embodiments, such as generally illustrated in FIG. 1, the input component 110 can be configured to receive information from one or more medical devices 120. Further, the input component 110 can receive sensor data 202 from one or more medical imaging devices 120. Sensor data 202 can include, but not limited to, operational data and ground truth data for the system 102 and connected medical imaging devices 120. In examples, medical imaging devices can include, but are not limited to, medical imaging devices such as, computed tomography (CT) devices, magnetic resonance imaging (MRI) devices, positron emission tomography (PET) devices, or any other device involved in the medical imaging process. Further, the input component 110 can receive sensor data from the one or more various connected medical imaging devices 120. For example and without limitation, sensor data 202 can include any variety of sensor parameter 122 including: raw sensor data, fan speed data, component temperature data, material degradation data, usage data, environmental data, performance data, or any other measurement that can indicate performance of the device and/or whether a repair should be conducted (e.g., up to M parameters).

In embodiments, such as where the medical imaging device 120 can be a gantry heater, sensor data 202 can include a heater outlet temperature, a first front fan speed, a second front fan speed, a high plenum temperature, and/or a low plenum temperature. For example, a normal operating region for a gantry heater can include a temperature between 26 and 24 degrees Celsius. Temperatures detected outside the normal operating region can indicate that the medical imaging device 120 should undergo maintenance or repair (such as to maintain normal operations). In embodiments, temperature values for the plenum that are lower than the acceptable threshold along with a low heater outlet temperature can correspond to a heater failure issue of the medical imaging device 120. Low or high fan speeds can be associated with one or more conditions for repair. For example and without limitation, a high first front fan speed and a high second front fan speed can correspond to a filter clogging issue of the medical imaging device 120. In other examples, a low first front fan speed and a low second front fan speed can correspond to one or more fan failures of the medical imaging device 120. One or more of a variety of other parameters correlating to a failure mode for one or more components of the medical imaging device 120 can be monitored in a similar manner for one or more failure modes.

With embodiments, such as generally illustrated in FIGS. 1 and 2, the non-limiting remote planned maintenance system 102 can utilize the sensor data 202 in combination with other data via a pre-processing component 204. The pre-processing component 204 can be operatively connected with the processor 106, the input component 110, the degradation component 112, and/or the planned maintenance component 114. In examples, the pre-processing component 204 can include logging one or more various sensor parameters (corresponding to the medical imaging devices 120) across tables in a data lake. Further, the non-limiting remote planned maintenance system 102 can fetch data through querying over time for the medical imaging devices 120. For instance, sensor data 202 can include data aggregation and merging one or more model's granularity 206. Sensor data 202 can include time series sensor data. Further, sensor data 202 can be collected/monitored over varying time frequencies and can be monitored over varying fixed time amounts (e.g., 4 hours, 8 hours, or 12 hours) and analyzed by the system 102. The sensor data 202 can be aggregated by the pre-processing component 204 such that the various tables of the table lake include a uniform time frequency for the various sensor parameters 122.

Such as generally illustrated in FIG. 2, the remote planned maintenance system 102 can integrate the various medical imaging device 120 sensor information via the pre-processing component 204. Additionally, the pre-processing component 204 can include a feature creation module 208 that can consider further sensor information. In examples, the feature creation module 208 can extract an additional set of features from the high frequency raw parameters (e.g., slope, moving average, moving standard deviation, time series decomposition using machine learning, frequency decomposition via wavelet-based techniques, etc.). The pre-processing component 204 can integrate the sensor data 202 with the additional set of features extracted via the feature creation module 208 to facilitate predicting and determining the planned maintenance of medical imaging devices 120.

Further, the feature creation module 208 can derive statistical and domain-based features from the sensor data 202. With examples, the feature creation module can determine mean, standard deviation, median, maximum, minimum, maximum to median ratio, min to median ratio, log transformation, polynomial transformations, and various other statistical operations. The feature creation module 208 can additionally consider physics-based features for the medical imaging devices 120. In examples, the feature creation module 208 can consider the rate of change of a sensor parameter with time, as well as the instant differences of the parameters (e.g., temperature, fan speed changes, performance measurements, etc.). Additionally, the feature creation module 208 can utilize a machine learning algorithm (e.g., decision tree, random forest, etc.) for the selection of features to be monitored.

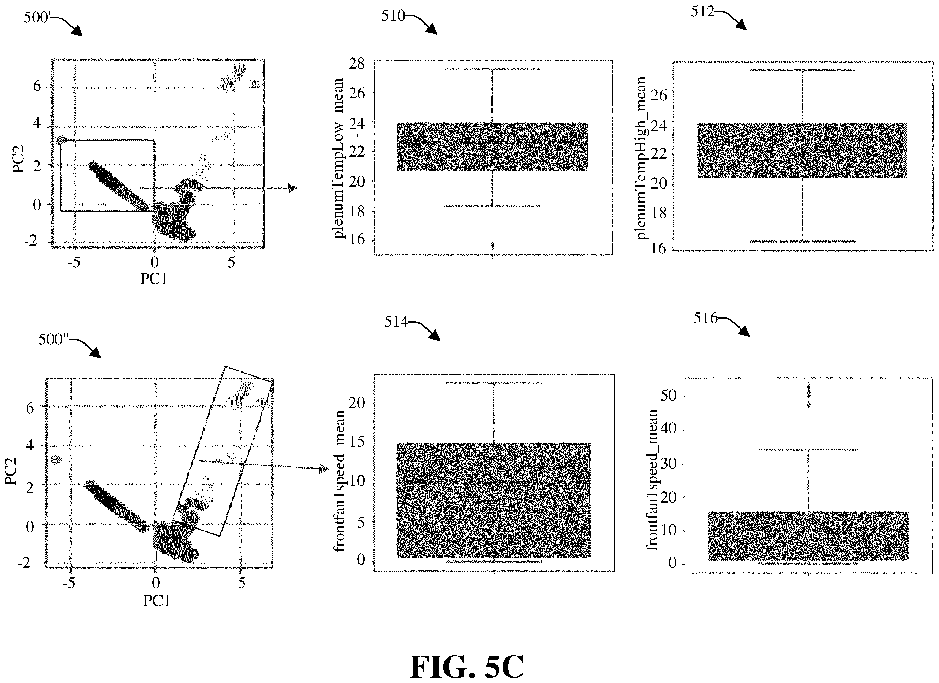

In embodiments, the remote planned maintenance system 102 (e.g., or the degradation component 112) can utilize a feature transformation algorithm 210 (e.g., an objective driven feature transformation algorithm) to further evaluate maintenance/performance of the medical imaging devices 120. The feature transformation algorithm 210 can transform the selected features of the medical imaging device 120 from a high dimensional feature space to a low dimensional feature space (e.g., where feature space can be a collection of features of the medical imaging device 120 that are further used to characterize data). Transformation can be achieved in one or more of a variety of algorithms/analysis, such as via Principal Component Analysis (e.g., and variations like Kernel Principal Component Analysis, Linear Discriminant Analysis, Facto Analysis, etc.). The resulting transformed features can enable the separation of a normal operating region for the medical imaging device 120 from a region of failure or degradation for the medical imaging device 120. The normal operating region and the failure regions for the medical imaging device 120 can be monitored so that the remote planned maintenance system 102 can determine whether maintenance should be performed on the medical imaging device 120 (e.g., or to postpone scheduled maintenance based on the acquired sensor data 202. The feature creation module 208 can utilize the above statistical information in determining the normal operating region and the failure/degradation regions for the medical imaging device 120. Further, the mean, standard deviation, and median (e.g., among other statistical operations) can be used by the feature transformation algorithm 210 to monitor and/or predict the performance of the medical imaging device 120 (such as generally illustrated in FIG. 5C).

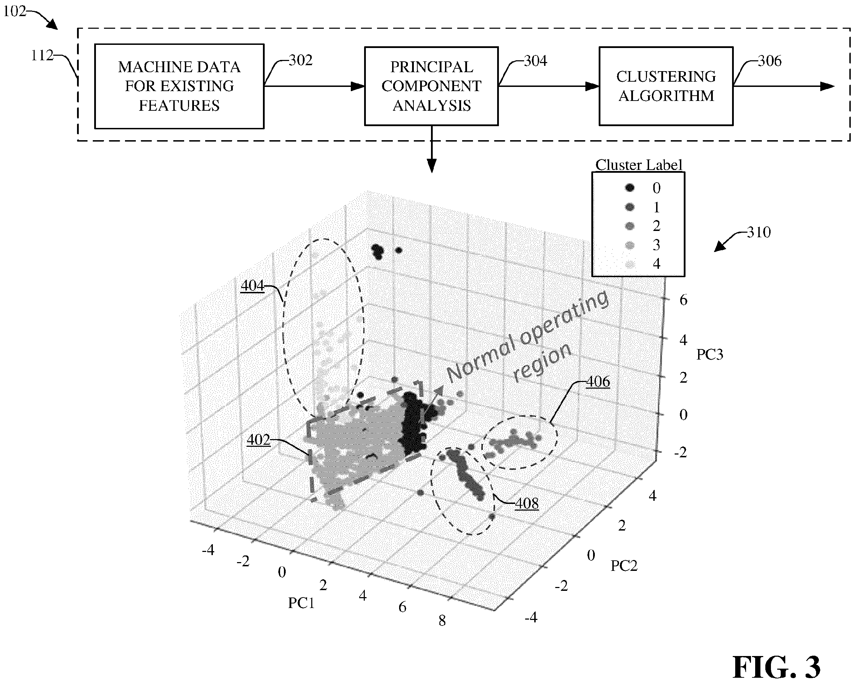

With embodiments, such as generally illustrated in FIG. 3, the degradation component 112 can generate a new set of variables that are uncorrelated in the transformed space (e.g., via the feature transformation algorithm 210). Further, boundaries for the normal operating region and failure regions of the medical imaging device 120 can be identified as shown by plot 310. For a medical imaging device 120 operating without abnormalities, it is expected to have the feature space reside within the defined operating region (e.g., normal operating region). Data points that deviate away from the normal operating region (e.g., which can be determined via statistical and physics-based features of the feature creation module 208) can indicate an emerging degradation in the medical imaging device 120. The normal operating region for a medical imaging device 120 can vary from device to device. Further, the normal operating region can be a single continuous region or can be one or more various disconnected regions based on the operation of the medical imaging device 120. In embodiments where regions of failure modes overlap, recommendations can be given to perform the planned maintenance tasks associated with each of the failure modes.

With embodiments, the non-limiting system 102 can separate fault data from normal operating data via principal component analysis 304 and applying a clustering algorithm 306. The degradation component 112 can further utilize machine data from existing features 302 and can employ principal component analysis 304 on the medical imaging device 120 to determine components of the medical imaging device 120 to be monitored or used in calculating a degradation of a medical imaging device 120. The degradation component 112 can divide the sensor data (e.g., and other calculated data) into one or more clusters.

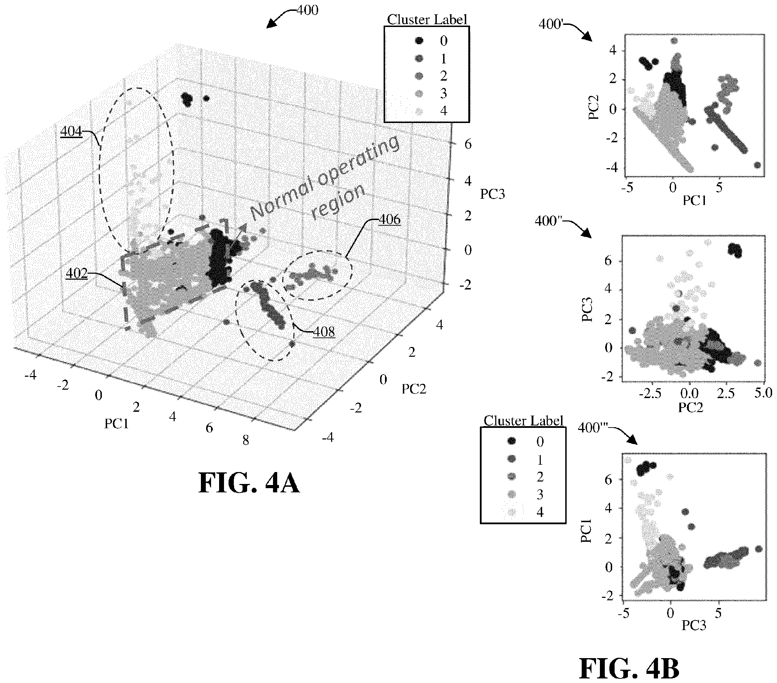

For example and without limitation, such as generally illustrated by FIG. 4A, the degradation component 112 can divide the sensor data 202 into a normal operating region 402, a first failure mode 404, a second failure mode 406, and a third failure mode 408. With embodiments, the first failure mode 404 can correspond to a heater failure (e.g., where the plenum temperature can be outside the acceptable range and the heater outlet temperature can be low), the second failure mode 406 can correspond to a filter clogging issue (e.g., where the first front fan speed and the second front fan speed can be high), and the third failure mode 408 can correspond to a fan failure of the medical imaging device 120 (e.g., where the first front fan speed and the second front fan speed can be low). The normal operating region 402 can correspond to a medical imaging device 120 functioning as intended (e.g., thereby not experiencing diminished performance). The failure modes 404-408 can correspond to specific planned maintenance tasks for the medical imaging device 120 (e.g., cleaning or replacing a fan/battery).

Such as illustrated in FIGS 4A and 4B, applying the principal component analysis 304 to the medical imaging device 120 can result in generating clusters for each of the component failures. In examples, for the failure modes, a boundary condition can be identified that defines the region of failure. The failure mode can be isolated in the transformed space and the current state of the medical imaging device 120 can be predicted based on the location of the datapoint in the associated plot 310. Boundaries of the clusters can be defined through algorithms such as K-means clustering, fuzzy clustering, density based clustering, and spectral clustering.

With embodiments, such as shown by FIGS. 4A and 4B, plot 400 represents a 3D clustering plot of principal components of the medical imaging device 120, as performed by the principal component analysis algorithm 304 and the clustering algorithm 306. The degradation component 112 can further generate one or more 2D clustering plots representative of the one or more failure modes for the associated medical imaging device 120 for further processing/analysis. 3D clustering plot 400 can be separated into a first 2D clustering plot 400', a second 2D clustering plot 400", and a third 2D clustering plot 400'". The first 2D clustering plot 400' can be utilized to determine whether the medical imaging device 120 is experiencing a heater failure. Such first 2D clustering plot 400' can be generated to analyze performance data associated with the plenum temperatures and the heater outlet temperature (e.g., to detect plenum temperatures outside of the acceptable range and a heater outlet temperature lower than the desired threshold indicating sub-optimal performance). The second 2D clustering plot 400" can be utilized to determine whether the medical imaging device 120 is experiencing filter clogging issues. Such second 2D clustering plot 400" can be generated to analyze performance data associated with the first front fan speed and the second front fan speed (e.g., to detect high fan speeds greater than the desired threshold indicating sub-optimal performance). The third 2D clustering plot 400'" can be utilized to determine whether the medical imaging device 120 is experiencing fan failures. Such third 2D clustering plot 400'" can be generated to analyze performance data associated with the first front fan speed and the second front fan speed (e.g., to detect low fan speeds lower than the desired threshold indicating sub-optimal performance).

In embodiments, the degradation component 112 can be configured to detect an emerging failure state. The degradation component 112 can predict the type of failure based on the location of the datapoint in the transformed space. For instance, datapoints approaching the boundaries of the normal operating region can indicate to the system 102 that an emerging failure state is likely and to schedule maintenance of the medical imaging device 120 (e.g., indicating a high propensity for failure, repair, or maintenance).

With embodiments, for datapoints lying outside the normal operating region 402, the deviation can be quantified through a degradation index. Further, the deviation can be calculated via a variety of methods (e.g., Euclidean distance, the projection of each datapoint from the center of the normal operating region, etc.). The quantified deviation can be scaled between a range of 0-100 to indicate the severity of the degradation for the associated failure. The degradation component can derive a threshold for the failure mode degradation indexes where components of the medical imaging device 120 are likely to fail (e.g., have a propensity to fail). For the failure modes 404-408, a degradation index 404', 406', 408' (e.g., such as seen in FIG. 3) can be calculated to analyze the degradation state of the medical imaging device 120. A higher degradation index can indicate a higher failure severity (where the medical imaging device 120 is more sensitive to failure and/or failure is more likely to result).

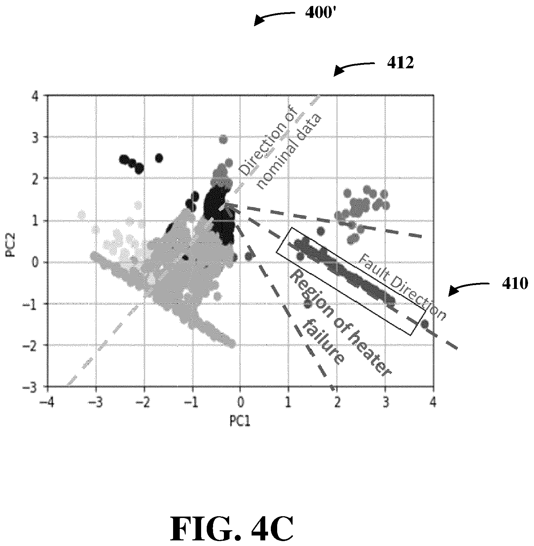

Turning now to FIG. 4C, the first 2D clustering plot 400' can be used to determine whether there is a heater failure of the medical imaging device 120; and further, whether maintenance should be performed. The failure modes 404-408 (e.g., the associated 2D clustering plots) can include a fault direction 410. The fault direction 410 an indicate that one or more components of the medical imaging device 120 includes performance data indicative of failure. For example and without limitation, the fault direction 410 includes values for components that lie outside the normal operating region 402. The planned maintenance of a medical imaging device 120 can be scheduled for components as performance values for such components of the medical imaging device 120 move away from a direction of nominal data 412.

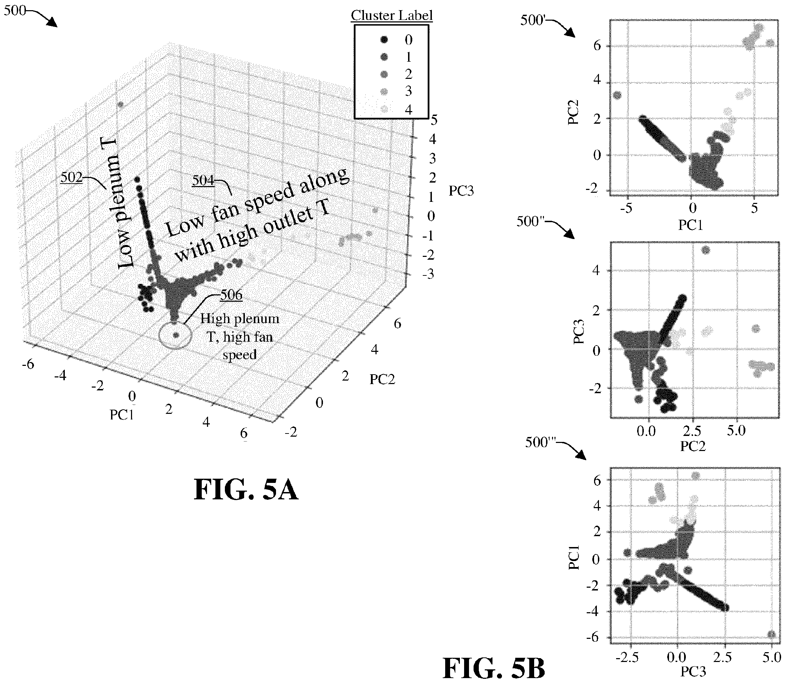

Such as generally illustrated in FIGS. 5A and 5B, the objective driven feature transformation algorithm 210 can employ Kernel Principal Component Analysis (PCA) 304 to generate a 3D clustering plot 500 of the principal components of the medical imaging device 120. The objective driven feature transformation algorithm 210 (e.g., and/or the degradation component 112) can further generate one or more 2D clustering plots representative of one or more failure modes for further processing/analysis. 3D clustering plot 500 can be separated into a first 2D clustering plot 500', a second 2D clustering plot 500", and a third 2D clustering plot 500'". A first failure mode 502 can correspond to a low plenum temperature; a second failure mode 504 can correspond to a low fan speed and high outlet temperature; and a third failure mode 506 can correspond to a high plenum temperature and a high fan speed.

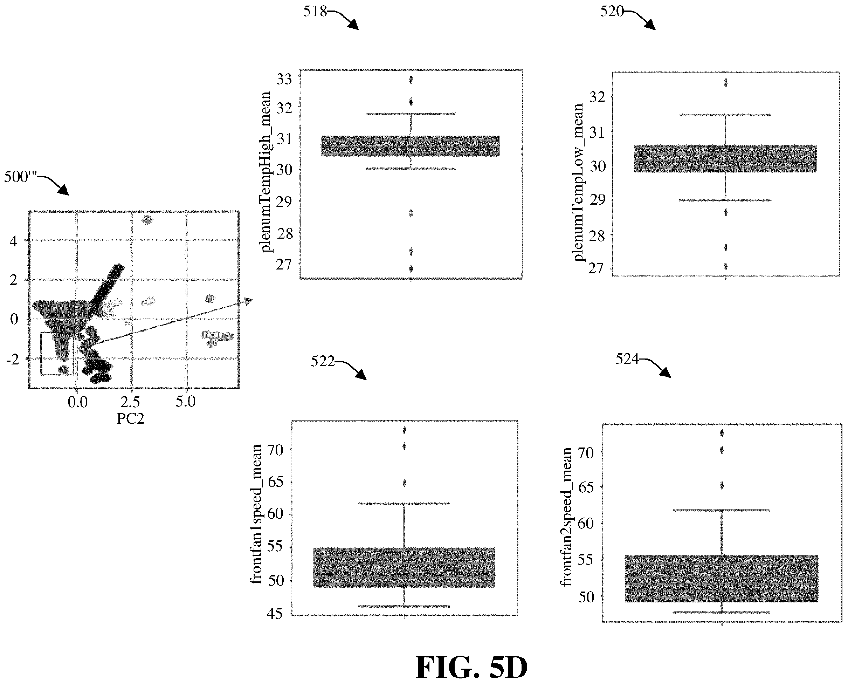

Turning next to FIGS. 5C and 5D, the degradation component 112 can utilize statistical and physics-based features from the feature creation module 208 of the pre-processing component 204 to determine whether the values of the components of the medical imaging device 120 are approaching the region of failure (e.g., where the component data is moving in a fault direction on the associated 2D plot). The 3D clustering plot 500 can be used to generate the first 2D clustering plot 500'; and further, the pre-processing component 204 (e.g., comprising data aggregation and merging one or more model's granularity 206 and the feature creation module 208) can statistically process the sensor data 202 to determine the mean of the low plenum temperature (shown by plot 510) and the mean of the high plenum temperature (shown by plot 512). In a similar manner, the pre-processing component 204 can additionally, statistically process the sensor data 202 to determine the mean of the first front fan speed (shown by plot 514) and the mean of the second front fan speed (shown by plot 516). As illustrated in FIG. 5D, the pre-processing component 204 can statistically process the sensor data 202 to determine the mean of the high plenum temperature (plot 518), the mean of the low plenum temperature (plot 520), the mean of the first front fan speed (plot 522), and the mean of the second front fan speed (plot 524).

In embodiments, the degradation component 112 can derive a threshold for each failure mode based on statistical or machine learning approaches (e.g., as executed by the pre-processing component 204). As the degradation index 404'-408' crosses the failure threshold of failure modes 404-408, the planned maintenance component 114 can generate a recommendation to a service engineer (e.g., remotely via a user interface 720) to perform the planned maintenance task associated with the failure modes 404-408 (see e.g., FIG. 4). Further, for overlapping regions of failure modes 404-408, where the degradation index is within the threshold, the service engineer can be asked to skip the associated planned maintenance task associated with the failure mode 404-408.

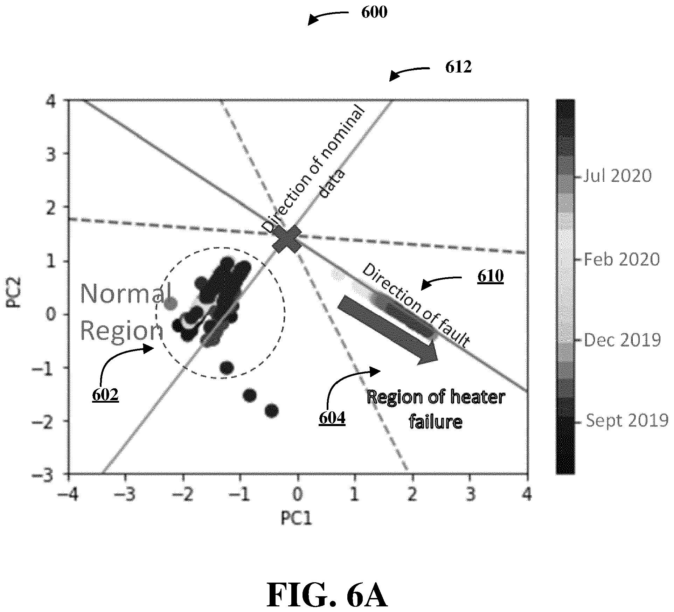

Such as generally illustrated in FIG. 6A, the degradation component 112 can measure a degree of separation between the normal operating region 602 and the degradation/failure mode region 604 by converting the separation into a degradation index (e.g., corresponding degradation indexes 404', 406', 408' such as in FIG. 2, or degradation index 620). The degradation index 620 can indicate the propensity for a particular failure mode (e.g., that a heater is likely to fail resulting in sub-optimal of the medical imaging device 120). Further, the degradation index 620 includes degradation scores linked to specific planned maintenance tasks, such as replacing a gantry heater. The degradation component 112 can utilize a root cause algorithm to determine the necessary repair based on the acquired sensor data 202 in combination with the degradation index 620. The root cause algorithm can be integrated with one or more repair manuals, reference manuals, root cause tables, and/or one or more maintenance algorithms to determine the necessary repair so that the medical imaging device 120 can return to optimal performance. The root cause algorithm can include information for a wide range of medical imaging devices 120 and components thereof to accurately diagnose and assess the root cause for the medical imaging device 120 failure.

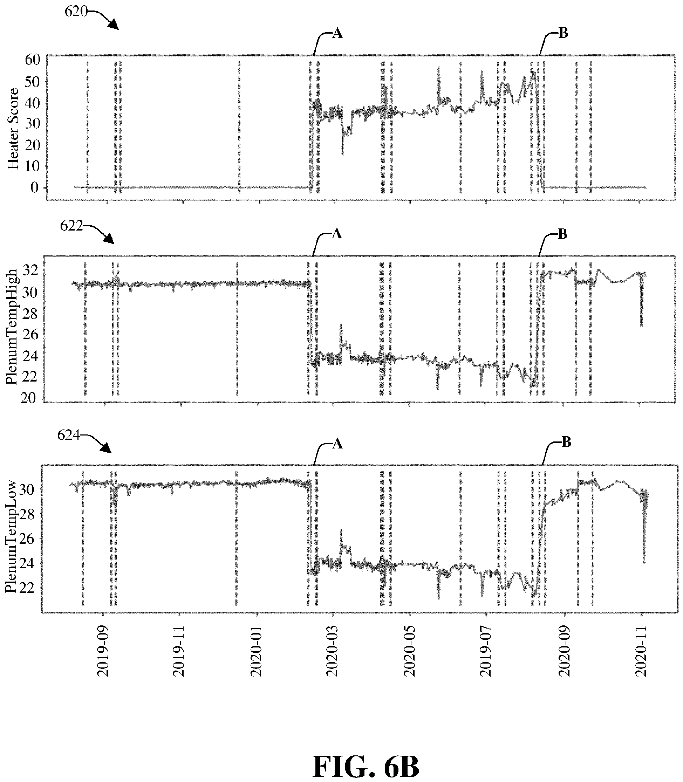

Further, with embodiments, the degradation index 620 can evaluate information from the high plenum temperature (plot 622) and the low plenum temperature (plot 624) during generation of the index. Such as can be seen from FIG. 6B, plot of degradation index 620 indicates a high degradation score for the gantry heater at point A on degradation index 620, and plots 622, 624 that can correspond to temperatures of the low plenum temperature and the high plenum temperature outside acceptable tolerances. Further, replacement of the gantry heater occurs at point B on degradation index 620, and plots 622, 624 resulting in the low plenum temperature and the high plenum temperature returning to a range of normal operation, and the degradation score of the gantry heater failure mode returning to a normal value (e.g., corresponding to the medical imaging device 120 operating normally and having correlating performance data indicating the same). Maintenance tasks including a high degradation score (e.g., a score that exceeds an acceptable performance threshold determined by the user and operating conditions of the medical imaging device 120) can be recommended to a service engineer via the user interface 720. For example, if the degradation score is greater than the desired threshold, such a score can indicate that the associated medical imaging device 120 includes a high failure sensitivity. Further, a high failure sensitivity can indicate that the medical imaging device 120 is likely to fail for one or more reasons (e.g., the medical imaging device 120 is likely to fail prior to the subsequent planned maintenance of the medical imaging device 120). In other embodiments, a high failure sensitivity can indicate that the performance of a component of the medical imaging device 120 is trending towards the fault direction for a particular failure region, thus maintenance can be required in urgent and non-urgent manners depending on how fast the component is trending towards the failure region.

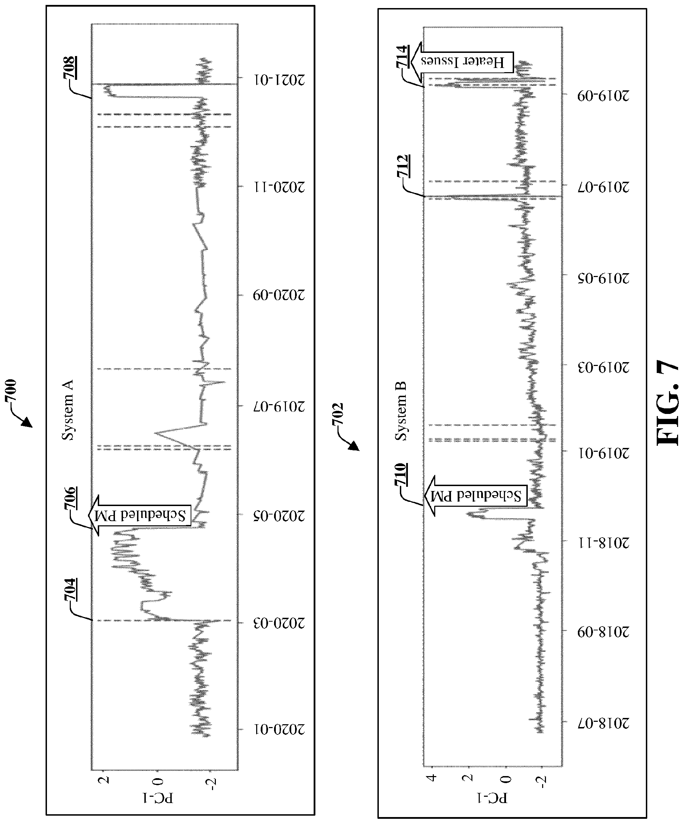

In examples, the planned maintenance component 114 can process the degradation index 620 via machine learning to determine a manner of planned maintenance for the various components of the medical imaging device 120 (e.g., the various failure modes/regions). Turning next to FIG. 7, the non-limiting remote planned maintenance system 102 can evaluate one or more degradation scores associated with failure modes of the medical imaging device 120. A first degradation plot 700 and a second degradation plot 702 can be generated for a failure mode (e.g., such as gantry heater failure) of the medical imaging device 120. Further, the first degradation plot 700 can include a first reference point 704 where the degradation score for the medical imaging device 120 increases indicating improper performance. In response, planned maintenance can be scheduled to correct performance of the medical imaging device 120 (e.g., at second reference point 706). The degradation score for heater failure can degrease and return to normalcy after replacing the heater via planned maintenance. In other embodiments, pre-scheduled maintenance can be waived if the degradation score indicates that the components of the associated medical imaging device are performing as expected. Additionally, degradation scores can return to normalcy without conducting pre-planned maintenance (such as at reference point 708), and such performance can be considered in scheduling the next planned maintenance.

With examples, in a similar manner with respect to first degradation plot 700, the second degradation plot 702 can include a first reference point 710 indicating scheduled and conducted planned maintenance in response to detecting high degradation scores. As can be seen from the second degradation plot 702, the degradation score for heater failure can decrease and returns to normalcy after conducting maintenance (e.g., via cleaning and/or component replacement). Further, a second reference point 712 can indicate a high degradation score returning to normalcy without planned maintenance. Additionally, a third reference point 714 can indicate a high degradation score due to heater failure whereby maintenance has not been pre-scheduled and maintenance should be performed to return the heater to optimal performance for the medical imaging device 120.

In examples, the first degradation plot 700 and the second degradation plot 702 can be visible within a user-interface 720 (as shown in FIG. 2). Further, the non-limiting remote planned maintenance system 102 can display one or more maintenance recommendations via the user-interface 720 that can be similar in appearance to plots 700 and 702.

For example and without limitation, planned maintenance can include any combination of the following actions: (1) powering down the medical imaging device 120, (2) powering down a component of the medical imaging device 120, (3) generating/transmitting an alert that the medical imaging device 120 is not functioning properly, (4) reducing performance of a component of the medical imaging device 120; (5) disconnecting the medical imaging device 120; (5) replacing a component (e.g., heat sink, fan, cooling fluid, bearings, and/or other properties of the components of the medical imaging device 120; and (6) replacing the medical imaging device 120. If maintenance has been scheduled for a medical imaging device 120, the remote planned maintenance system 102 can selectively disconnect components of the medical imaging device 120 and/or the medical imaging device 120 in whole to facilitate a safe and efficient repair process. For example, the remote planned maintenance system 102 can disconnect power to the medical imaging device 120 and await maintenance/repair. In addition, the remote planned maintenance system 102 can generate one or more alerts that the medical imaging device 120 will disconnect and/or will be inoperable until maintenance can be conducted (which can be remotely scheduled).

The user-interface 720 can be a checklist or a report that includes visuals (e.g., supporting charts, graphs, data, etc.) for service and/or field engineers to consider when conducing maintenance. The user interface 720 can additionally include a description of the current state of the components corresponding with the identified failure modes for the medical imaging device 120 (e.g., which can include highlights to direct service engineers to important information on the user interface 720). For failure modes where the degradation index is high, the user interface 720 can display a root cause for the high degradation index. The user planned maintenance component 114 can employ a root cause algorithm in identifying the root cause for a failure mode, thus integrating the degradation index. The user interface 720 can be connected on site with the various medical imaging devices 120 such that a service engineer can easily access and interact with the user interface 720.

In embodiments, the non-limiting remote planned maintenance system 102 can be evaluated by one or more various metrics. For example and without limitation, such metrics can include a recall percentage and a percentage of planned maintenance tasks avoided. The recall percentage can be expressed as:

$$\frac{True\ Positives}{True\ Positives + False\ Negatives}$$

. The recall percentage can reflect the percentage of failures/errors/issues that are detected of the medical imaging device 120. Further, false negatives can represent situations where the remote planned maintenance system 102 misses prediction of a defective component for the associated medical imaging device 120. True positives can represent situations where the component is defective and the remote planned maintenance system 102 has accurately predicted and/or diagnosed such failure. Additionally, the percentage of planned maintenance tasks avoided can be expressed as:

$$\frac{Number\ of\ planned\ maintenance\ tasks\ avoided}{Total\ planned\ maintenance\ tasks}$$

. The precision of the non-limiting remote planned maintenance system 102 can assessed and expressed as

$$\frac{True\ Positives}{True\ Positives + False\ Positives}$$

(e.g., where false positives can be instances where a failure/error/issue is detected, however does not actually occur). An increased precision of the remote planned maintenance system 102 can be associated with reduced false calls (failure calls) for the medical imaging device 120. An increased recall percentage of the remote planned maintenance system 102 can be associated with an increased rate of capture of failures for the medical imaging device 120.

With embodiments, a computer-implemented method of remote planned maintenance 800 can comprise a first step of receiving, using a processor operatively coupled to memory, sensor data 202 from at least one medical imaging device 120 (step 802). The computer implemented method can further include generating, using the processor, cluster regions indicating failure modes and a normal operating mode for the medical imaging device 120 (step 804). In generating the cluster regions indicating failure and normal operating modes, the method can include the pre-processing component 204 (e.g., the feature creation module 808) deriving statistical and domain-based features from the sensor data 202 (step 806). Further, the feature creation module can determine statistical operations of the sensor data 202 for processing the operating regions for the medical imaging device 120. In such a step, the feature creation module 208 can identify the components that contribute to successful performance of the medical device 120 and transmit the identified components to the objective driven feature transformation algorithm 210.

Additionally, the computer implemented method 800 can include utilizing, using the processor, a feature transformation algorithm 210 to determine a degradation score for a planned maintenance task of the at least one medical imaging device 120 based on the sensor data 202 (step 808). Further, the feature transformation algorithm 210 can be utilized to transform the selected features of the medical imaging device 120 from a high dimensional feature space to a low dimensional feature space (e.g., for more efficient processing and/or data analysis). The feature transformation algorithm 210 can transform the data such that the degradation component 112 can determine the performance of the medical imaging device 120 by comparing historical and current operation of the components with the normal operating region and the failure operating regions. Statistical operations utilized by the feature transformation algorithm 210 can be used to monitor and/or predict the performance of the medical imaging device 120 with respect to the normal operating region and failure regions (that are to be determined by the method 800).

In embodiments, the computer-implemented method of remote planned maintenance 800 can include determining thresholds for components for the failure modes/regions of the medical imaging device 120. Thresholds for failure modes/regions can be determined by applying the clustering algorithm 306. For example and without limitation, the method 800 can include determining thresholds for failure modes of the medical imaging device 120 by generating a 3D clustering plot via the principal component analysis algorithm 304 and the clustering algorithm 306. Further, the degradation component 112 can generate one or more resulting 2D cluster plots from the 3D clustering plot reflective of the one or more failure modes/regions for the medical imaging device 120. The degradation component can accurately assess performance and/or maintenance of the medical imaging device 120 via the one or more resulting 2D cluster plots.

With embodiments, the degradation component can determine the severity of datapoints (e.g., sensor data 202) lying outside of the normal operating region. The severity can be determined via the deviation from the normal operating region. Further, datapoints that are farther away from the normal operating region can indicate a higher degree of sub-optimal performance (e.g., a likelihood of medical imaging device 120 failure).

The remote planned maintenance method 800 can include determining whether the degradation score of a failure mode is greater than the threshold (e.g., for repair) (step 810). If the degradation score is not greater than the threshold, the planned maintenance component 114 can provide no recommendations for planned maintenance (step 812). For example and without limitation, if the threshold is not exceeded, the planned maintenance component 114 can recommend not performing (e.g., skipping) the planned maintenance for the medical imaging device 120. With embodiments, planned maintenance tasks can be checked off a checklist (e.g., having any number of planned maintenance tasks) for the associated medical imaging device 120 by the planned maintenance component 114. Further, tasks can be checked of a list instead of being conducted. Conversely, if the degradation score is greater than the threshold, the planned maintenance component 114 can transmit an alert to the user interface 720 and can recommend maintenance for the associated component of the failure mode corresponding to the high degradation score (e.g., a degradation score that exceeded a limit set by the user or by the functionalities of the specific medical imaging device 120) (step 814). In examples, the user interface 720 can include any variety of informational medium to effectively communicate an alert (e.g., graphs, tables, visualizations, data, root cause, etc.). Further, in transmitting an alert, the planned maintenance component 114 can perform one or more of a variety of actions. The planned maintenance component 114 can disconnect the medical imaging device 120 from the power source, selectively disconnect one or more components of the medical imaging device 120 from the power source, and remotely reduce performance of the medical imaging device 120 (e.g., enable a medical device to be in a lower power mode to reduce component operational demand). The planned maintenance component 114 can generate an alert while the device is isolated from further damage (e.g., disconnected from power and/or operation) and awaiting repair.

In order to provide additional context for various embodiments described herein, FIG. 9 and the following discussion are intended to provide a brief, general description of a suitable computing environment 900 in which the various embodiments of the embodiment described herein can be implemented. While the embodiments have been described above in the general context of computer-executable instructions that can run on one or more computers, those skilled in the art will recognize that the embodiments can be also implemented in combination with other program modules and/or as a combination of hardware and software.

Generally, program modules include routines, programs, components, data structures, etc., that perform particular tasks or implement particular abstract data types. Moreover, those skilled in the art will appreciate that the various methods can be practiced with other computer system configurations, including single-processor or multiprocessor computer systems, minicomputers, mainframe computers, Internet of Things (IoT) devices, distributed computing systems, as well as personal computers, hand-held computing devices, microprocessor-based or programmable consumer electronics, and the like, each of which can be operatively coupled to one or more associated devices.

The illustrated embodiments herein can be also practiced in distributed computing environments where certain tasks are performed by remote processing devices that are linked through a communications network. In a distributed computing environment, program modules can be located in both local and remote memory storage devices.

Computing devices typically include a variety of media, which can include computer-readable storage media, machine-readable storage media, and/or communications media, which two terms are used herein differently from one another as follows. Computer-readable storage media or machine-readable storage media can be any available storage media that can be accessed by the computer and includes both volatile and nonvolatile media, removable and non-removable media. By way of example, and not limitation, computer-readable storage media or machine-readable storage media can be implemented in connection with any method or technology for storage of information such as computer-readable or machine-readable instructions, program modules, structured data, or unstructured data.

Computer-readable storage media can include, but are not limited to, random access memory (RAM), read only memory (ROM), electrically erasable programmable read only memory (EEPROM), flash memory or other memory technology, compact disk read only memory (CD-ROM), digital versatile disk (DVD), Blu-ray disc (BD) or other optical disk storage, magnetic cassettes, magnetic tape, magnetic disk storage or other magnetic storage devices, solid state drives or other solid state storage devices, or other tangible and/or non-transitory media which can be used to store desired information. In this regard, the terms "tangible" or "non-transitory" herein as applied to storage, memory, or computer-readable media, are to be understood to exclude only propagating transitory signals per se as modifiers and do not relinquish rights to all standard storage, memory or computer-readable media that are not only propagating transitory signals per se.

Computer-readable storage media can be accessed by one or more local or remote computing devices, e.g., via access requests, queries, or other data retrieval protocols, for a variety of operations with respect to the information stored by the medium.

Communications media typically embody computer-readable instructions, data structures, program modules or other structured or unstructured data in a data signal such as a modulated data signal, e.g., a carrier wave or other transport mechanism, and includes any information delivery or transport media. The term "modulated data signal" or signals refers to a signal that has one or more of its characteristics set or changed in such a manner as to encode information in one or more signals. By way of example, and not limitation, communication media include wired media, such as a wired network or direct-wired connection, and wireless media such as acoustic, RF, infrared, and other wireless media.

With reference again to FIG. 9, the example environment 900 for implementing various embodiments of the aspects described herein includes a computer 902, the computer 902 including a processing unit 904, a system memory 906 and a system bus 908. The system bus 908 couples system components including, but not limited to, the system memory 906 to the processing unit 904. The processing unit 904 can be any of various commercially available processors. Dual microprocessors and other multi-processor architectures can also be employed as the processing unit 904.

The system bus 908 can be any of several types of bus structure that can further interconnect to a memory bus (with or without a memory controller), a peripheral bus, and a local bus using any of a variety of commercially available bus architectures. The system memory 906 includes ROM 910 and RAM 912. A basic input/output system (BIOS) can be stored in a nonvolatile memory such as ROM, erasable programmable read only memory (EPROM), EEPROM, where BIOS contains the basic routines that help transfer information between elements within the computer 902, such as during startup. The RAM 912 can also include a high-speed RAM such as static RAM for caching data.

The computer 902 further includes an internal hard disk drive (HDD) 914 (e.g., EIDE, SATA), one or more external storage devices 916 (e.g., a magnetic floppy disk drive (FDD) 916, a memory stick or flash drive reader, a memory card reader, etc.) and an optical disk drive 920 (e.g., which can read or write from a CD-ROM disc, a DVD, a BD, etc.). While the internal HDD 914 is illustrated as located within the computer 902, the internal HDD 914 can also be configured for external use in a suitable chassis (not shown). Additionally, while not shown in environment 900, a solid-state drive (SSD) could be used in addition to, or in place of, an HDD 914. The HDD 914, external storage device(s) 916 and optical disk drive 920 can be connected to the system bus 908 by an HDD interface 924, an external storage interface 926 and an optical drive interface 928, respectively. The interface 924 for external drive implementations can include at least one or both of Universal Serial Bus (USB) and Institute of Electrical and Electronics Engineers (IEEE) 1394 interface technologies. Other external drive connection technologies are within contemplation of the embodiments described herein.

The drives and their associated computer-readable storage media provide nonvolatile storage of data, data structures, computer-executable instructions, and so forth. For the computer 902, the drives and storage media accommodate the storage of any data in a suitable digital format. Although the description of computer-readable storage media above refers to respective types of storage devices, it should be appreciated by those skilled in the art that other types of storage media which are readable by a computer, whether presently existing or developed in the future, could also be used in the example operating environment, and further, that any such storage media can contain computer-executable instructions for performing the methods described herein.

A number of program modules can be stored in the drives and RAM 912, including an operating system 930, one or more application programs 932, other program modules 934 and program data 936. All or portions of the operating system, applications, modules, and/or data can also be cached in the RAM 912. The systems and methods described herein can be implemented utilizing various commercially available operating systems or combinations of operating systems.

Computer 902 can optionally comprise emulation technologies. For example, a hypervisor (not shown) or other intermediary can emulate a hardware environment for operating system 930, and the emulated hardware can optionally be different from the hardware illustrated in FIG. 9. In such an embodiment, operating system 930 can comprise one virtual machine (VM) of multiple VMs hosted at computer 902. Furthermore, operating system 930 can provide runtime environments, such as the Java runtime environment or the .NET framework, for applications 932. Runtime environments are consistent execution environments that allow applications 932 to run on any operating system that includes the runtime environment. Similarly, operating system 930 can support containers, and applications 932 can be in the form of containers, which are lightweight, standalone, executable packages of software that include, e.g., code, runtime, system tools, system libraries and settings for an application.

Further, computer 902 can be enabled with a security module, such as a trusted processing module (TPM). For instance, with a TPM, boot components hash next in time boot components, and wait for a match of results to secured values, before loading a next boot component. This process can take place at any layer in the code execution stack of computer 902, e.g., applied at the application execution level or at the operating system (OS) kernel level, thereby enabling security at any level of code execution.

A user can enter commands and information into the computer 902 through one or more wired/wireless input devices, e.g., a keyboard 938, a touch screen 940, and a pointing device, such as a mouse 942. Other input devices (not shown) can include a microphone, an infrared (IR) remote control, a radio frequency (RF) remote control, or other remote control, a joystick, a virtual reality controller and/or virtual reality headset, a game pad, a stylus pen, an image input device, e.g., camera(s), a gesture sensor input device, a vision movement sensor input device, an emotion or facial detection device, a biometric input device, e.g., fingerprint or iris scanner, or the like. These and other input devices are often connected to the processing unit 904 through an input device interface 944 that can be coupled to the system bus 908, but can be connected by other interfaces, such as a parallel port, an IEEE 1394 serial port, a game port, a USB port, an IR interface, a BLUETOOTH® interface, etc.

A monitor 946 or other type of display device can be also connected to the system bus 908 via an interface, such as a video adapter 948. In addition to the monitor 946, a computer typically includes other peripheral output devices (not shown), such as speakers, printers, etc.

The computer 902 can operate in a networked environment using logical connections via wired and/or wireless communications to one or more remote computers, such as a remote computer(s) 950. The remote computer(s) 950 can be a workstation, a server computer, a router, a personal computer, portable computer, microprocessor-based entertainment appliance, a peer device or other common network node, and typically includes many or all of the elements described relative to the computer 902, although, for purposes of brevity, only a memory/storage device 952 is illustrated. The logical connections depicted include wired/wireless connectivity to a local area network (LAN) 954 and/or larger networks, e.g., a wide area network (WAN) 956. Such LAN and WAN networking environments are commonplace in offices and companies, and facilitate enterprise-wide computer networks, such as intranets, all of which can connect to a global communications network, e.g., the Internet.

When used in a LAN networking environment, the computer 902 can be connected to the local network 954 through a wired and/or wireless communication network interface or adapter 958. The adapter 958 can facilitate wired or wireless communication to the LAN 954, which can also include a wireless access point (AP) disposed thereon for communicating with the adapter 958 in a wireless mode.

When used in a WAN networking environment, the computer 902 can include a modem 960 or can be connected to a communications server on the WAN 956 via other means for establishing communications over the WAN 956, such as by way of the Internet. The modem 960, which can be internal or external and a wired or wireless device, can be connected to the system bus 908 via the input device interface 944. In a networked environment, program modules depicted relative to the computer 902 or portions thereof, can be stored in the remote memory/storage device 952. It will be appreciated that the network connections shown are example and other means of establishing a communications link between the computers can be used.

When used in either a LAN or WAN networking environment, the computer 902 can access cloud storage systems or other network-based storage systems in addition to, or in place of, external storage devices 916 as described above. Generally, a connection between the computer 902 and a cloud storage system can be established over a LAN 954 or WAN 956 e.g., by the adapter 958 or modem 960, respectively. Upon connecting the computer 902 to an associated cloud storage system, the external storage interface 926 can, with the aid of the adapter 958 and/or modem 960, manage storage provided by the cloud storage system as it would other types of external storage. For instance, the external storage interface 926 can be configured to provide access to cloud storage sources as if those sources were physically connected to the computer 902.

The computer 902 can be operable to communicate with any wireless devices or entities operatively disposed in wireless communication, e.g., a printer, scanner, desktop and/or portable computer, portable data assistant, communications satellite, any piece of equipment or location associated with a wirelessly detectable tag (e.g., a kiosk, news stand, store shelf, etc.), and telephone. This can include Wireless Fidelity (Wi-Fi) and BLUETOOTH® wireless technologies. Thus, the communication can be a predefined structure as with a conventional network or simply an ad hoc communication between at least two devices.

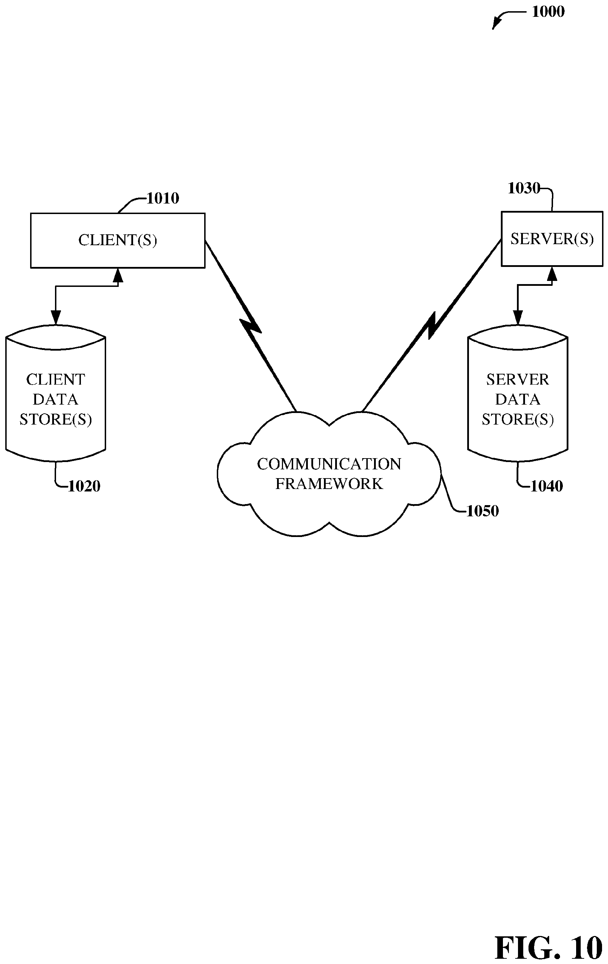

Referring now to FIG. 10, there is illustrated a schematic block diagram of a computing environment 1000 in accordance with this specification. The system 1000 includes one or more client(s) 1002, (e.g., computers, smart phones, tablets, cameras, PDA's). The client(s) 1002 can be hardware and/or software (e.g., threads, processes, computing devices). The client(s) 1002 can house cookie(s) and/or associated contextual information by employing the specification, for example.

The system 1000 also includes one or more server(s) 1004. The server(s) 1004 can also be hardware or hardware in combination with software (e.g., threads, processes, computing devices). The servers 1004 can house threads to perform transformations of media items by employing aspects of this disclosure, for example. One possible communication between a client 1002 and a server 1004 can be in the form of a data packet adapted to be transmitted between two or more computer processes wherein data packets may include coded analyzed headspaces and/or input. The data packet can include a cookie and/or associated contextual information, for example. The system 1000 includes a communication framework 1006 (e.g., a global communication network such as the Internet) that can be employed to facilitate communications between the client(s) 1002 and the server(s) 1004.

Communications can be facilitated via a wired (including optical fiber) and/or wireless technology. The client(s) 1002 are operatively connected to one or more client data store(s) 1008 that can be employed to store information local to the client(s) 1002 (e.g., cookie(s) and/or associated contextual information). Similarly, the server(s) 1004 are operatively connected to one or more server data store(s) 1010 that can be employed to store information local to the servers 1004.

In one exemplary implementation, a client 1002 can transfer an encoded file, (e.g., encoded media item), to server 1004. Server 1004 can store the file, decode the file, or transmit the file to another client 1002. It is noted that a client 1002 can also transfer uncompressed file to a server 1004 and server 1004 can compress the file and/or transform the file in accordance with this disclosure. Likewise, server 1004 can encode information and transmit the information via communication framework 1006 to one or more clients 1002.

The illustrated aspects of the disclosure may also be practiced in distributed computing environments where certain tasks are performed by remote processing devices that are linked through a communications network. In a distributed computing environment, program modules can be located in both local and remote memory storage devices.

The above description includes non-limiting examples of the various embodiments. It is, of course, not possible to describe every conceivable combination of components or methods for purposes of describing the disclosed subject matter, and one skilled in the art may recognize that further combinations and permutations of the various embodiments are possible. The disclosed subject matter is intended to embrace all such alterations, modifications, and variations that fall within the spirit and scope of the appended claims.

With regard to the various functions performed by the above-described components, devices, circuits, systems, etc., the terms (including a reference to a "means") used to describe such components are intended to also include, unless otherwise indicated, any structure(s) which performs the specified function of the described component (e.g., a functional equivalent), even if not structurally equivalent to the disclosed structure. In addition, while a particular feature of the disclosed subject matter may have been disclosed with respect to only one of several implementations, such feature may be combined with one or more other features of the other implementations as may be desired and advantageous for any given or particular application.

The terms "exemplary" and/or "demonstrative" as used herein are intended to mean serving as an example, instance, or illustration. For the avoidance of doubt, the subject matter disclosed herein is not limited by such examples. In addition, any aspect or design described herein as "exemplary" and/or "demonstrative" is not necessarily to be construed as preferred or advantageous over other aspects or designs, nor is it meant to preclude equivalent structures and techniques known to one skilled in the art. Furthermore, to the extent that the terms "includes," "has," "contains," and other similar words are used in either the detailed description or the claims, such terms are intended to be inclusive - in a manner similar to the term "comprising" as an open transition word - without precluding any additional or other elements.

The term "or" as used herein is intended to mean an inclusive "or" rather than an exclusive "or." For example, the phrase "A or B" is intended to include instances of A, B, and both A and B. Additionally, the articles "a" and "an" as used in this application and the appended claims should generally be construed to mean "one or more" unless either otherwise specified or clear from the context to be directed to a singular form.

The term "set" as employed herein excludes the empty set, i.e., the set with no elements therein. Thus, a "set" in the subject disclosure includes one or more elements or entities. Likewise, the term "group" as utilized herein refers to a collection of one or more entities.

The description of illustrated embodiments of the subject disclosure as provided herein, including what is described in the Abstract, is not intended to be exhaustive or to limit the disclosed embodiments to the precise forms disclosed. While specific embodiments and examples are described herein for illustrative purposes, various modifications are possible that are considered within the scope of such embodiments and examples, as one skilled in the art can recognize. In this regard, while the subject matter has been described herein in connection with various embodiments and corresponding drawings, where applicable, it is to be understood that other similar embodiments can be used or modifications and additions can be made to the described embodiments for performing the same, similar, alternative, or substitute function of the disclosed subject matter without deviating therefrom. Therefore, the disclosed subject matter should not be limited to any single embodiment described herein, but rather should be construed in breadth and scope in accordance with the appended claims below.

## Claims

1. A system comprising:

   a memory that stores computer executable components; and
   a processor, operably coupled to the memory, and that executes the computer executable components stored in the memory, wherein the computer executable components comprise:

   an input component that receives sensor data from at least one medical device;
   a degradation component that utilizes a feature transformation algorithm to determine a degradation score for a planned maintenance task of the at least one medical device based on the sensor data; and
   a planned maintenance component that recommends maintenance for the at least one medical device based on the degradation score.

2. The system of claim 1, wherein the degradation score indicates a propensity for a failure mode of the at least one medical device.

3. The system of claim 2, wherein the planned maintenance component determines a failure threshold for the planned maintenance task, and degradation scores greater than the failure threshold indicate required maintenance for the at least one medical device.

4. The system of claim 2, wherein the degradation component extracts statistical and domain-based features from the sensor data; and transforms the statistical and domain-based features to a feature space enabling separation between a normal operating region and a failure region of the at least one medical device.

5. The system of claim 4, wherein the degradation score is based on a degree of separation between the normal operating region and the failure region for the at least one medical device.

6. The system of claim 3, wherein the degradation score is calculated from a current degradation and a history of degradation values of the at least one medical device.

7. The system of claim 6, wherein if the degradation score is greater than the failure threshold, a root cause is determined by the degradation component utilizing a root cause algorithm.

8. The system of claim 3, wherein the planned maintenance component performs an action in response to determining that the at least one medical device requires maintenance; and the action includes at least one of disconnecting the at least one medical device from power, transmitting an alert that the at least one medical device is at risk of failure, and reducing performance of the at least one medical device.

9. A computer implemented method for enabling remote planned maintenance of medical devices using an algorithm, comprising:

   receiving, using a processor operatively coupled to memory, sensor data from at least one medical device;
   utilizing, using the processor, a feature transformation algorithm to determine a degradation score for a planned maintenance task of the at least one medical device based on the sensor data; and
   recommending, using the processor, maintenance for the at least one medical device based on the degradation score.

10. The computer implemented method of claim 9, wherein the degradation score indicates a propensity for a failure mode of the at least one medical device.

11. The computer implemented method of claim 10, further comprising:
   determining, using the processor, a failure threshold for the planned maintenance task, wherein degradation values greater than the failure threshold indicate required maintenance for the at least one medical device.

12. The computer implemented method of claim 11, further comprising:

extracting, using the processor, statistical and domain-based features from the sensor data; and
transforming, using the processor, the statistical and domain-based features to a feature space enabling separation between a normal operating region and a failure region of the at least one medical device.

13. The computer implemented method of claim 12, wherein the degradation score is based on a degree of separation between the normal operating region and the failure region for the at least one medical device.

14. The computer implemented method of claim 11, wherein the degradation score is calculated from a current degradation and a history of degradation values of the at least one medical device.

15. The computer implemented method of claim 14, further comprising:

   determining, using the processor, a root cause for the at least one medical device if the degradation score is greater than the failure threshold; and
   displaying, using the processor, the root cause for the at least one medical device to a user.

**FIG. 1**

**FIG. 2**

EP 4 372 508 A1

FIG. 3

EP 4 372 508 A1

FIG. 4B

FIG. 4A

FIG. 4C

**FIG. 5A**

**FIG. 5B**

EP 4 372 508 A1

**FIG. 5C**

FIG. 5D

EP 4 372 508 A1

FIG. 6A

**FIG. 6B**

**FIG. 7**

800

802

RECEIVING, USING A PROCESSOR OPERATIVELY COUPLED TO MEMORY, SENSOR DATA FROM AT LEAST ONE MEDICAL DEVICE

804

GENERATING, USING THE PROCESSOR, CLUSTER REGIONS INDICATING FAILURE MODES AND A NORMAL OPERATING MODE FOR THE MEDICAL DEVICE

806

DERIVING, USING THE PROCESSOR, STATISTICAL AND DOMAIN BASED FEATURES FROM THE SENSOR DATA

808

UTILIZING, USING THE PROCESSOR, A FEATURE TRANSFORMATION ALGORITHM TO DETERMINE A DEGRADATION SCORE FOR A PLANNED MAINTENANCE TASK OF THE AT LEAST ONE MEDICAL DEVICE BASED ON THE SENSOR DATA

810

DEGRADATION SCORE OF FAILURE MODE > THRESHOLD

812

NO RECOMMENDATION FOR PLANNED MINTENANCE

814

OUTPUT TO USER INTERFACE, RECOMMEND MAINTENANCE FOR COMPONENT ASSOCIATED WITH FAILURE MODE > THRESHOLD

**FIG. 8**

**FIG.9**

1000

1010

CLIENT(S)

1030

SERVER(S)

CLIENT
DATA
STORE(S)

1020

COMMUNICATION
FRAMEWORK

1050

SERVER
DATA
STORE(S)

1040

FIG. 10

Europäisches
Patentamt
European
Patent Office
Office européen
des brevets

# EUROPEAN SEARCH REPORT

Application Number

EP 23 20 7109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/148718 A1 (SCHRIVER RALPH [US] ET AL) 12 May 2022 (2022-05-12) * The whole document, in particular: Paragraphs: [0050] – [0054], [0077] – [0086], [0092], [0099] – [0110]. Figures: Fig. 2, Fig. 3. * | 1-15 | INV. G05B23/02 G06F11/00 G06Q10/20 G16H40/40 |
| A | MELANI ARTHUR HENRIQUE DE ANDRADE ET AL: "A framework to automate fault detection and diagnosis based on moving window principal component analysis and Bayesian network", RELIABILITY ENGINEERING AND SYSTEM SAFETY., vol. 215, 1 November 2021 (2021-11-01), page 107837, XP093142888, GB ISSN: 0951-8320, DOI: 10.1016/j.ress.2021.107837 Retrieved from the Internet: URL:https://pdf.sciencedirectassets.com/271430/1-s2.0-S0951832021X00087/1-s2.0-S0951832021003574/main.pdf> [retrieved on 2024-03-15] * The whole document, in particular: Pages: 2, 4. * | 1-15 | |

TECHNICAL FIELDS SEARCHED (IPC)

G05B
G06Q
G16H
G06F

-/--

The present search report has been drawn up for all claims

| Place of search | Date of completion of the search | Examiner |
|---|---|---|
| Munich | 20 March 2024 | Zacharias, Malte |

Europäisches
Patentamt
European
Patent Office
Office européen
des brevets

# EUROPEAN SEARCH REPORT

Application Number

EP 23 20 7109

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SHAMAYLEH ABDULRAHIM ET AL: "IoT Based Predictive Maintenance Management of Medical Equipment", JOURNAL OF MEDICAL SYSTEMS, SPRINGER US, NEW YORK, vol. 44, no. 4, 20 February 2020 (2020-02-20), XP037028615, ISSN: 0148-5598, DOI: 10.1007/S10916-020-1534-8 [retrieved on 2020-02-20] * The whole document, in particular: Page: 4. * | 1-15 | |
| A | US 2020/185085 A1 (MAVRIEUDUS DIMITRIOS [NL] ET AL) 11 June 2020 (2020-06-11) * The whole document, in particular: Paragraphs: [0007] – [0013], [0024] – [0027], [0035], [0036], [0039] – [0046], [0052]. * | 1-15 | |

TECHNICAL FIELDS
SEARCHED (IPC)

The present search report has been drawn up for all claims

| Place of search | Date of completion of the search | Examiner |
|---|---|---|
| Munich | 20 March 2024 | Zacharias, Malte |

EPO FORM 1503 03.82 (P04C01)

EP 4 372 508 A1

## ANNEX TO THE EUROPEAN SEARCH REPORT
## ON EUROPEAN PATENT APPLICATION NO.

EP 23 20 7109

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2024

| Patent document cited in search report | | Publication date | Patent family member(s) | | Publication date |
|---|---|---|---|---|---|
| US 2022148718 | A1 | 12-05-2022 | AU | 2018317866 A1 | 23-01-2020 |
| | | | CA | 3068658 A1 | 21-02-2019 |
| | | | EP | 3669373 A1 | 24-06-2020 |
| | | | JP | 7200145 B2 | 06-01-2023 |
| | | | JP | 2020531940 A | 05-11-2020 |
| | | | JP | 2023029403 A | 03-03-2023 |
| | | | US | 2020118675 A1 | 16-04-2020 |
| | | | US | 2022148718 A1 | 12-05-2022 |
| | | | WO | 2019035986 A1 | 21-02-2019 |
| US 2020185085 | A1 | 11-06-2020 | CN | 110870022 A | 06-03-2020 |
| | | | EP | 3652748 A1 | 20-05-2020 |
| | | | JP | 7333276 B2 | 24-08-2023 |
| | | | JP | 2020526816 A | 31-08-2020 |
| | | | US | 2020185085 A1 | 11-06-2020 |
| | | | WO | 2019011765 A1 | 17-01-2019 |

EPO FORM P0459

For more details about this annex : see Official Journal of the European Patent Office, No. 12/82